# EUROPEAN PATENT SPECIFICATION

(11) **EP 1 386 302 B1**
(45) Date of publication and mention of the grant of the patent: **21.06.2006**
(21) Application number: 02712974.1
(22) Date of filing: 28.03.2002
(51) Int. Cl.: G08C 25/00, A61B 5/00

(54) **ARRANGEMENT FOR REGISTRATION**
ANORDNUNG ZUR REGISTRATION
ENSEMBLE D'ENREGISTREMENT

(30) Priority: 28.03.2001 FI 20010640; 30.10.2001 FI 20012100
(43) Date of publication of application: 04.02.2004
(73) Proprietor: INCREA OY, 00530 Helsinki (FI)
(72) Inventor: SEPPONEN, Raimo, FIN-00530 Helsinki (FI)
(74) Representative: LEITZINGER OY
(86) International application number: PCT/FI2002/000271
(87) International publication number: WO 2002/084624

(56) References cited:
- EP-A2- 0 371 261
- WO-A1-02/41773
- US-A- 5 370 496
- PATENT ABSTRACTS OF JAPAN & JP 07 334 783 A (NIPPON BARUUFU KK) 22 December 1995

## Description

The invention relates an arrangement including means, which may be used for registration of signals, such as EEG, ECG and EMG, from one or several objects, such as human body, animal, apparatus or equipment, or from apparatus or equipment signal or signals such as signals including information about temperature, pressure or sound and for transmit via local or wide area networks information related to signals, for exploit information related to signals in control of operations and to control operations related to registration of signals

In the following an object means generally an object, from which one will register some signals and/or which is accompanied by operational means, which may be controlled and in controlling one may exploit information obtained from the signals registered from the object.

So, the object may be a human, an animal, a device or equipment.

In practice there exists situations, when one wants to register signals from an object and information obtained from these signals is exploited in various ways. In instrumentation, in which a transducer and units connected to that are at different electrical potential referenced to each other and to surroundings, there will appear common mode currents due to voltage differences. As a pathway for a common mode current serves often in addition to a conductor or conductors for utility signal at least one or several pathway often including couplings through stray capacitances between units and surroundings. An example of such a pathway is the safety ground of equipment. These common mode currents cause electromagnetic interference in many registrations of signals and in control of operational means. A strong common mode current and associated common mode voltage may damage equipment and may be harmful to a user or to an object under study.

Often there are several signals to be registered. As an example of such an object is a patient, from which one during a surgical operation may monitor function of heart using ECG and blood pressure BP. In addition one may monitor level of muscle relaxation using EMG. Depth of anesthesia one may monitor using electric signal from brain, EEG. Level of oxygen saturation of blood one may monitor using pulse oximetry. Corresponding monitoring is carried out also in emergency departments and in various combinations in wards. As the health care at homes becomes more popular this kind of monitoring is implemented also at homes and nursing homes. A necessary instrumentation for signal registration and patient monitoring is manufactured among others by Instrumentarium Ltd, Datex-Ohmeda Division, Helsinki, Finland.

Basics of signal registration are discussed in a book: J.G. Webster (Editor): Medical Instrumentation Application and Design, Third Edition, John Wiley & Sons, New York 1998. In this book in pages 233-286 one has discussed registration of biopotentials and related problems. The electrical safety issues has been discussed in pages 623-658. Because of safety reasons and to decrease electrical interference one applies often isolation amplifiers, which are manufactured among others Analog Devices Inc., USA and Burr Brown Inc., USA. Registration is associated by interferences either from object or surroundings. On the other hand a registration itself may interfere with some other activity. One such situation is magnetic resonance imaging. During magnetic resonance imaging one applies electromagnetic irradiation at the resonance frequency on the object, which usually is a human body. This frequency is about 42 MHz when field strength of a magnetic resonance imaging unit is 1 T and as a target nucleus is the nucleus of hydrogen atom i.e. the proton. When the strength of the magnetic field is 3 T, the proton resonance frequency is about 130 MHz. Magnetic resonance imaging methods and devices are described among others in the publication Sepponen RE: US5592084, to which and to the references included are referred herein.

The excitation pulses during magnetic resonance imaging have a high peak power, even several kilowatts, and they are repeated hundreds times during an imaging process. If one wants to register e.g. electric signal from brain, i.e. EEG-signal, these pulses and for imaging needed gradient pulses generate easily interferences. Due to a high magnetic field strength a movement of a patient, even due to beating of the heart, generates noise voltages in cables.

A current flowing through electrodes may generate burn injuries in tissues below electrodes. Because of this one attempts to isolate the registration devices from surroundings using various solutions. These include among others various optical, wireless and ultrasound solutions, which are described in references US4737712, US4763075, US5323776, US5394873, US5445162, US5733247, US6032063, and US6198287. These are referenced as a prior art of technology. Another environment where interferences at several megahertz exist is an operation theatre, where applied electrical knives generate strong interferences and hence a good isolation and noise suppression capability of amplifiers is to be expected. In ambulances applied radiophones may disturb registration of biosignals, such as ECG. Such strong noise sources are GSM and TETRA phones. Therefore use of radiophones in hospitals is not allowed, especially in sites where the reliability of operation of instrumentation is important. Correspondingly use of phones is not allowed in airplanes and their use is expected to be limited in industrial and in other sites where a reliable operation of instrumentation is important.

In X-ray devices, especially in so-called intraoral devices intended for imaging of intraoral objects such as teeth- or jaw tissues, one has been started to utilize e.g. CCD or CMOS-based transducers. From these one must transfer information fast to a central unit, so that the radiographer may verify succeeding of the image and evaluate a potential need for further imaging. To the transducer one must provide energy for operation and in addition one must transfer a high rate signal to the central unit. Because of safety the transducer must be electrically isolated from surroundings. In addition from the X-ray source a noise voltage, generated by the high voltage generator of the X-ray tube, may be coupled. This may be solved by using wireless transfer between the transducer and the central unit and by providing energy of operation with battery or accumulator. This solution has problems of a large size of the battery and potential disturbances in the wireless transmission, which may lead to a bad image and in this way to a need for rescanning and hence to unnecessary irradiation load of a patient.

There are several practical problems with present solutions: Although optical isolation is effective to prevent coupling of electromagnetic energy it is technically often an awkward solution. One must provide energy for the isolated section using batteries, accumulators or light cells, which have a poor power transform ratio. A modulation of light signal is also complex and because of this the number of channels remains small and it is not easy to increase or decrease. Isolation with a transform coupling works well only at low frequencies, because there is a large capacitance between primary and secondary windings. Isolation amplifiers are manufactured among others by Burr Brown Inc. and Analog Devices Inc., USA.

The problem with present solutions is, how one may advantageously provide isolation, over which one transfer both signal and power without the front stage to posses a significant capacitance with surroundings. To minimize this capacitance is important for noiseless registration of a signal and for safety, as described earlier and in the references mentioned above. Present solutions are prone to a large number of cables. This is a clear disadvantage among others with patient monitoring and especially with monitoring during surgical operation. Corresponding problems are present also with other instrumentation arrangements such as with industrial, car and airplane electronics.

With an arrangement of the invention it is possible to solve such problems as described earlier.

An arrangement of the invention is characterized by what is set forth in the characterizing sections of the annexed claims.

The invention is illustrated in the accompanying drawings,
FIG 1 shows a present way to realize a multiparameter registration, as an example a medical instrumentation system.
Fig 2 shows a way of realization according to the invention.
Fig 3 shows an arrangement of the invention at a block diagram level.
Fig 4 shows a registration unit of an arrangement of the invention at a block diagram level.
Fig 5 shows a use of an arrangement of the invention with a magnetic resonance imaging unit.
FIG 6 shows an arrangement of the invention at a block diagram level.
FIG 7 shows an arrangement of the invention for registration of multichannel EEG, i.e. electric signal from brain
FIG 8 shows an alternative realization of an isolation section.
FIG 9 shows a realization of an arrangement of the invention, which may be applied e.g. in registration of NMR-signal from an interior of a human body.
FIG 10 shows an application of an arrangement of the invention in patient monitoring and in association with a structure of a patient bed.
FIG 11 shows an arrangement of the invention realized with clothing
FIG 12 shows a realization of a connector, which may be applied in an arrangement of the invention.

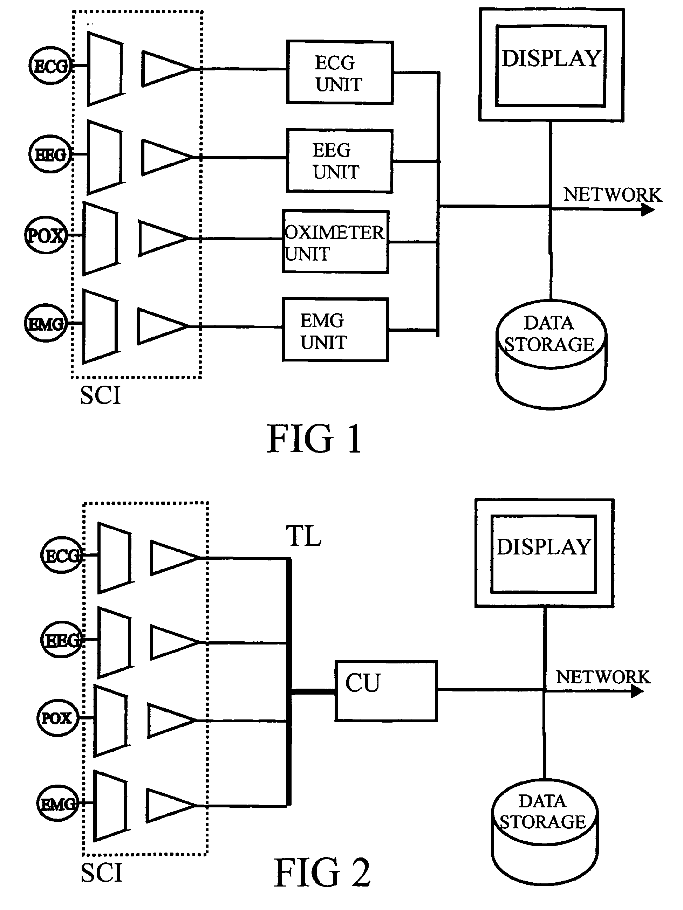

FIG 1 shows a present way to realize a multiparameterregistration. In this example the parameters are ECG, i.e. electric signal from heart, EEG, i.e. electric signal from brain, POX, i.e. measurement of oxygen saturation level of blood with pulse oximetry, and EMG, i.e. electric signal from muscle. These are signals which are registered e.g. from a patient during surgical operation. According to the present practice for each measurand one has devoted a dedicated signal conditioning unit, SCI (signal conditioning + isolation), including an isolation, through which the processed signals are conducted to signal registration unit for each signal, ECG UNIT, EEG UNIT, OXIMETER UNIT, EMG UNIT. From these the signals may be conducted to display unit, DISPLAY or to storage unit, DATA STORAGE units and eventually transmitted to data network, NETWORK. Often the registration units are modules, which may be applied as many as needed in an equipment rack. In the equipment rack there is a central processor and connections to display units, keyboards, and data networks. These types of systems are manufactured among others by Instrumentarium Ltd/Datex-Ohmeda Division (Finland), as mentioned earlier. The signals registered by registration units may be conducted to a specific central unit, which may include signal processing and monitoring of limit values and may give for example alarm signals. Disadvantages of the solution described above are a significant capacitance, which may lead to coupling of interference e.g, during surgical operation performed with an electric knife and that a large number of patient cables is required.

Fig 2 shows one multiple parameter registration system of the invention corresponding to the example shown in FIG 1. The signals are conducted to the central unit, CENTRAL UNIT via a common transmission line, TL. This line has two tasks: through that to the signal conditioning unit go power transmitted by the CENTRAL UNIT and received signals transmitted by the signal conditioning unit. CENTRAL UNIT may also control operation of various parts of a signal conditioning unit and these signals travel along transmission line. SCI includes means for signal processing and isolation.

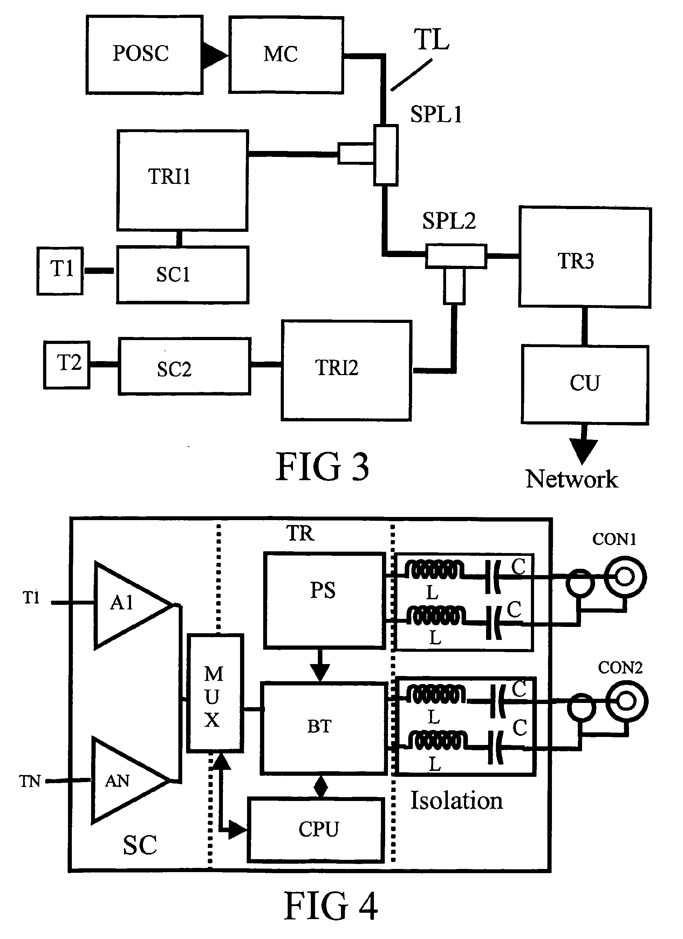

FIG 3 shows an arrangement of the invention for registration of two signals. The signals are registered with transducers T1 and T2. These are connected to signal processing means, SC 1 and SC2. SC 1 and SC2 are connected to connection circuits, which include isolation means TRI1 and TRI2. Via these isolation means both transfer of power and signals takes place. In the following signal conditioning means SC1 and SC2 and connection means TRI1 and TRI2 are unanimously called signal means. In the transmission line TL there are two connection circuits SPL1 and SPL2 (splitter). The central unit CU is connected to transmission line TL via transmission/receiver circuit TR3. TR3 does not necessarily include isolation. Power is being fed at least to the transducer and signal means T1, T2, SC1, SC2, TRI1 and TRI2 from the power oscillator POSC (power oscillator) and matching means MC (matching circuit) and through transmission line TL. The operating frequency of POSC is high, e.g. over 1 GHz, compared to that of significant electromagnetic interferences appearing in surroundings. Transmission and receiving circuits may be realized using so called Bluetooth technique. Circuits related to this are produced by several manufacturers, e.g. Ericsson AB, Sweden. POSC may be realized using components, which are applied in mobile phones. E.g. circuits used for registration of biosignals need very small driving power, so for a system of many measurands most often one watt driving power is enough. When needed, for example during sensitive measurement activities, POSC may operate in periods. In this case too it is advantageous, if signal produced by POSC has a narrow bandwidth.

FIG 4 shows at block diagram level one possible realisation of signal conditioning unit connected to transducer, transmission/receiving unit and isolation. Signals, which may be obtained from transducers T1 and TN will be amplified and eventually filtered with circuits A1 and AN. There may be as many transducers T1, TN as needed. The processor unit CPU, which may be realized using a proper microcontroller, controls a multiplexing circuit MUX, which may be realised using ready-made microcircuits and analog switches, which are available from various manufacturers. MUX may include an analog-to-digital converter. Circuit BT has been realised using a Bluetooth - transceiver circuit. All mentioned circuits get their power from rectifier and stabilizing circuit PS (power supply), which may include a switching mode power supply circuit, which is used to convert power from connection CON1 (connection) to driving power. Power may be conducted to connection CON1 through a coaxial cable. The figure shows that the isolation has been realised using serial connection of two capacitors C and inductors L. When the power is introduced at 2,4 GHz frequency and C is 2 pF, then the impedance of C is about -j33 Ω. This may be compensated with an inductor of 2,2 nH. Then C and L have a serial resonance with about zero impedance.

This serial connection has an impedance of about 0,5 kΩ at 3 T proton resonance frequency of 120 MHz and about 3 kΩ at an operating frequency of an electric knife (under 30 MHz). Then safety risks are minimal and interferences are small. At mains frequency of 50 or 60 Hz the impedance is over 1 GΩ hence by mains voltage generated leakage currents are minimal. Leakage currents may be further minimized by reduction of value of C and by compensating this by increasing the value of L correspondingly. However, in practice the capacitance of cables and transducers and especially that of the object under study to surroundings is markedly larger, therefore a reduction of C has not an essential significance. An input impedance of the power supply circuit must be matched with the impedance of the transmission line attached to CON1 so that power would be transferred as effectively as possible. Typically an impedance of a coaxial cable is 50 or 75 Ω. Because of this PS includes necessary means and this technology is known from the field of radio technology. Correspondingly similar isolation is between BT and CON2 connection. Although in this solution one has thought to connect to CON1 for example a coaxial cable or some other transmission line, to this may be connected also an antenna and wirelessly transmit signals of BT. In fact, in hospital, with magnetic resonance imaging, and in many industrial environments it is not desirable to use wireless connection because of interferences. If one connects BT to antenna isolation is not necessarily needed but it is desirable because the antenna may make an accidental contact with surroundings.

Examples of alternative realizations:

In some simple cases one may exploit in signal transfer amplitude-, frequency-, phase-, or pulse ratio modulation and use in realisation of transmit/receive circuits TRI, TRI2, TR3 a microcircuit TRF 6900A and as transmit-only circuit TRF 4900, which both are manufactured by Texas Instruments, USA. The operation voltage is 2,2 - 3,6 V and the need for operation current is low. These circuits are packaged in small 24 and 48 pins TSSOP and PQFP cases. If acquired as bare chips their size is really small and they may be bonded directly to the bonding pad using a proper bonding technology. In this solution the power oscillator POSC may operate for example at frequency of 2,4 GHz, for this frequency operation there are low cost components available.

If one is willing to operate at higher frequency, for example because of isolation, one may exploit as the transmit/receive circuit the circuit AR7501A operating at 5 - 6 GHz frequency and manufactured by Araftek Inc., California, USA.

Bluetooth is just one usable standard solution. On the market there are also other suitable standards such as e.g. IEEE 802.11, which is under development for a version capable to reach rate of 22 Mbit/s. Even higher data transfer speed may be reached using so-called OFDM-solutions (OFDM: Orthogonal Frequency Division Multiplexing). OFDM is based on a use of several parallel frequencies in data transfer and hence it uses frequency band more effectively than for example conventional spread spectrum techniques.

The frequency at which power is transferred should be selected in such a way that one may use so small capacitive coupling with isolation ISOLATION that those electromagnetic energies coupled to object P and in following to be called significant energies and which may cause electromagnetic interferences in signals and may pose a significant safety risk when coupled via the registration devices to surroundings, are prevented to flow via isolation ISOLATION so that their disturbing effect on signal or on increase of a safety risk remains insignificant. This means in practice that for a frequency of power transfer one should select a frequency, which is markedly higher than the highest frequency of the coupled significant electromagnetic energy. Generally a frequency difference of one decade is suffcient to provide enough attenuation. Hence, e.g. with 3 T magnetic resonance imaging it is feasible to use a transfer frequency higher than 1 GHz.

Isolation may be realized also with a transformer, which may be custom made or one may use ready-made transformer components, which are manufactured among others by Mini Circuits, USA.

The transducers T1, T2, TN may also be passive, such as electrodes or active, such as for example strain gages, pressure transducers, linear transformers, and Hall transducers. In most cases these will get their power via isolation ISOLATION. The signal conditioning means SC may also be active or passive. An example of a passive SC is a simple low pass filter made from a resistor and a capacitor. For every implementation of the invention means SC are not necessary.

Because a transfer of energy via isolation ISOLATION is demanding, it is often desirable to keep the power requirement of the electronics behind isolation as small as feasible.

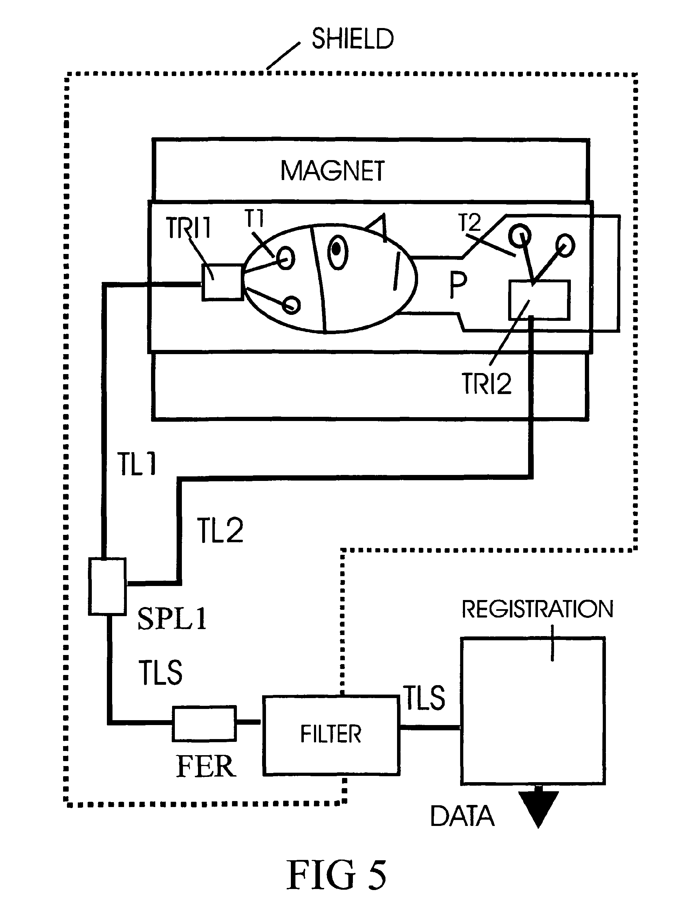

FIG 5 shows diagrammatic presentation of a registration arrangement of the invention. An object P (person) is under a study in a magnetic resonance imaging device, from which the Figure 5 shows only a magnet, MAGNET, and a surrounding shielded room SHIELD. During scanning various signals from the body of P such as ECG, EEG, EMG, pulse oximetry, respiration, movement of eyes, blood pressure, respiration gases. This registration is performed with transducers T1 and T2, which may be for example skin electrodes. T1 and T2 are connected to registration units TRI1 and TRI2. TRI1 and TRI2 are connected via transmission lines TL1 and TL2 (Transmission line) to the dividing circuit SPL1, which is further connected to transmission line TLS, which further passes through a filter FILTER the wall of the shielded room SHIELD and is connected to a main registration unit REGISTRATION, which further transmits processed data DATA forward if necessary. The figure shows also that there is a ferrite FER on the transmission line TLS, the purpose of this is to attenuate common mode interferences coupled along the transmission line TLS. In an arrangement of the invention one may exploit ferrite components in many ways to improve compatibility properties because a transmission line is advantageously realised in such a way that it includes conductors for signal and return currents, like for example a coaxial cable.

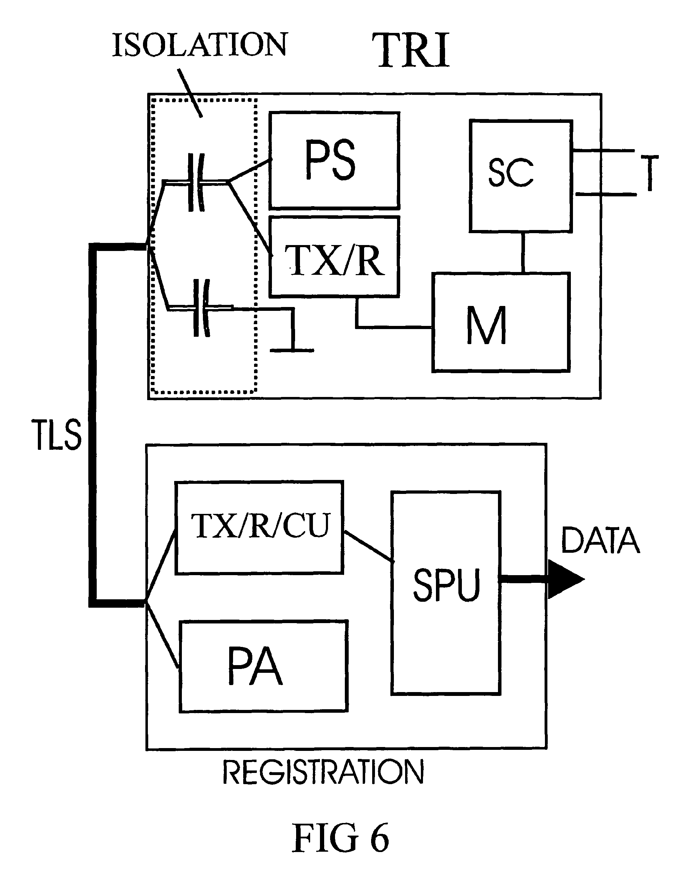

FIG 6 depicts in more detail operation of one arrangement of the invention. REGISTRATION unit includes a power amplifier PA (Power Amplifier), which is connected to transmission line TLS and feeds in it electromagnetic energy e.g. at frequency of 2,4 GHz. A proper, small-sized, stable but still relatively powerful (even 800 mW) integrated circuit PM2117 is manufactured by Pacific Monolithics Inc, Sunnyvale, California, USA. Because this frequency is about 20 times higher than e.g. the proton resonance frequency at 3T of a magnetic resonance imaging unit one may realize isolation in the registration unit simply by using capacitors, for which there is a dedicated space ISOLATION in TRI. From energy, which passes through ISOLATION TRI takes driving energy, this is taken care by the power supply unit PS (Power Supply).

It should be mentioned, that isolation may be realized in some other part of an arrangement. Isolation may be distributed in the transmission line, e.g. in the cable there is a section, where the energy transfer takes place capacitively. As an isolation means is suitable also a transformer and some combination of transformer and capacitor. Via same transmission line one may transfer also a registered signal. For this purpose TRI includes a TX/R unit. It should be noted, that this unit may also include a receiver R (Receiver). With this one may receive for example control and calibration information. TRI includes also a signal circuit SC (Signal Conditioning), which processes signals obtained from transducers T for a modulator M, which drives TX/R unit. As modulation one may use e.g. so called spread spectrum technique. It is possible that a large part of the operation of the equipment is realized using so called Bluetooth technique, which is designed mainly for wireless local area networks. For this purpose TRI includes matching circuits to match TLS to TX/R circuits, TLS may be a coaxial cable, which prevents effectively any coupling of driving power and signal energies to surroundings. It should be emphasized that although a signal transfer could be done in a wireless manner, it is most desirable in this application keep the radio frequency energy inside the transmission line. Such environments are emergency departments and intensive care units in hospitals.

Instead of the spread spectrum technique one may also use so called ultra wide bandwidth modulation. In this technique signal modulation consists of very short pulses and the bandwidth is several gigahertz, typically 1 - 3 GHz. In this case signal resembles noise in time space and it does not markedly disturb other equipment.

REGISTRATION unit includes correspondingly TX/R/CU unit for transmitting and receiving of signals. These signals are conducted to signal processing unit SPU (Signal Processing Unit), from where they are transmitted forward as processed information DATA. It should be mentioned that DATA may be simple status or alarm information or more complex real-time signal, which is conducted to display means or may be exploited in control e.g. operation of a magnetic resonance imaging unit. One such a kind of operation is synchronization of an imaging event with functional phases of heart of an object (Cardiac Gating). DATA may be a part of a study of mapping of function of brain. In this type of a study DATA is combined with information provided by functional magnetic resonance imaging. The applications of the invention are not limited in control of magnetic resonance imaging, but it may be applied also in control of operation of other diagnostic or therapeutic measures. These include for example brain surgery, control of anesthesia, and control of rehabilitation.

The FIG. 3 shows, how it is possible with an arrangement of the invention to give variety to a measurement system. With splitters one may connect several transmission lines TL1, TL2 and to these several registration units RT1 and RT2 respectively.

To sum up the benefits of the invention:

Through a transmission line one may conduct needed driving energy and by selecting the transmission frequency to be clearly higher than the signal and noise frequencies, one may realize the needed isolation in a simple manner, for example using capacitors. In a solution of the invention common mode currents propagating along the transmission line remain small because of the isolation. Respectively interferences generated in registration are minimized. The same transmission line may be used also to transfer signal information to the central unit. By using the energy to be transmitted one may with a proper modulation, e.g. using spread spectrum modulation, reach a high information transfer capability. In addition by using splitters one may divide a transmission line for several registration units. Depending on the modulation technique there may be several units. Cables suitable for the application are manufactured by W.L. Gore and Associates and Micro-Coax Inc. USA. Suitable components for signal dividing between different transmission lines are manufactured by Mini Circuits Inc. USA. Bluetooth components are manufactured by among others Ericsson AB, Sweden. Bluetooth modules, which are suitable for applications of the invention, are manufactured also among others Taiyo Yuden, Japan. This manufacturer produces a module suitable for applications in mobile phones and also in applications of the invention. The size of the said module is 15 x 14 x 2,2 mm. The manufacturer has also a module having USB and UART connections and a FLASH memory. This module is suitable as the transmit/receive circuit TR3 in FIG 3. Transmission lines with splitters may be implemented in a frame of a patient bed. Another advantageous equipment of medical technology, in which the transmission lines are feasible to implement at least partially in the frame, is an anesthesia machine. These applications bring forward one advantage of the invention, which is a reduction of a number of needed cables.

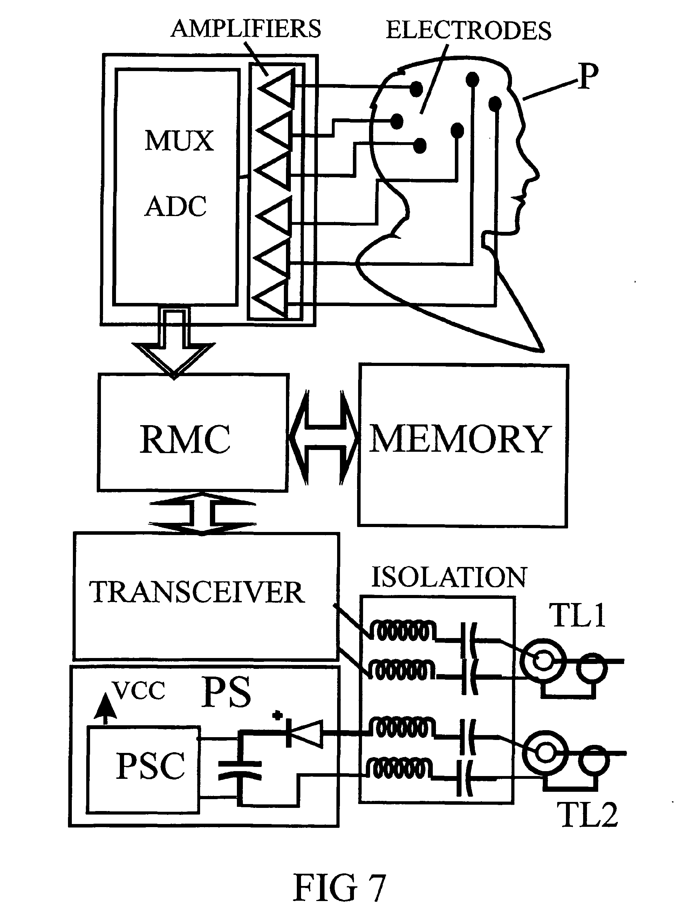

FIG 7 shows one solution of the invention, where from an object P electrical activity of brain is to be registered in a multichannel manner. On the object P there are attached electrodes ELECTRODES, which are connected to amplifiers AMPLIFIERS. These are connected via a multiplexing circuit MUX to analog/digital converter ADC. From ADC circuit signal is conducted to processor RMC (registration microcontroller), which stores necessary information in memory means MEMORY, which may be a semiconductor memory, for example so called FLASH memory. From the processor circuit RMC information is conducted to transmission and modulation means TRANSCEIVER, which may be accordant with the Bluetooth concept. Power supply voltage VCC for this whole device is provided by the power supply means PS (Power Supply), which may include regulator means PSC (Power Supply Controller).

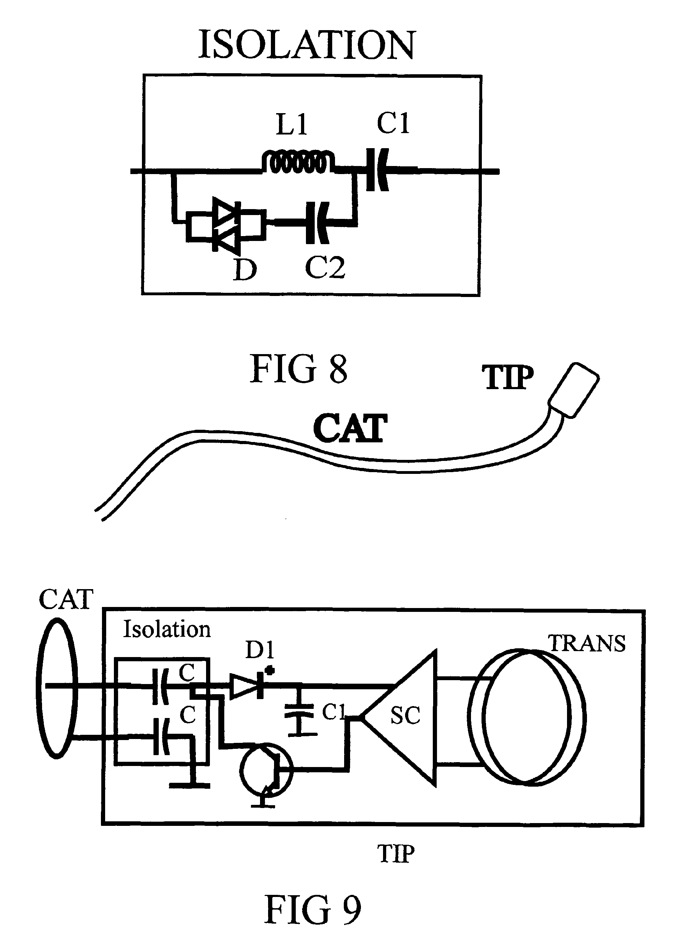

The means described above are connected to transmission lines TL1 and TL2 via isolation means ISOLATION. As shown in FIG 5, the means ISOLATION may include a capacitive C1 and an inductive L1 element. The values of these have been set so, that at the frequency of information and power they form a series resonance, which has low impedance. Because excitation power used in magnetic resonance imaging is high, it may be considered necessary to increase the impedance of isolation specifically at the excitation frequency. This may be effected simply by adding a suitable capacitance C2 parallel to the series inductance as shown in FIG 8. As a result said inductance and said capacitance form a parallel resonance circuit with a high impedance. A pair of diodes D may be connected in series with the capacitance, this prevents a coupling of the capacitance if the signal level is not high. The transfer capacity of Bluetooth is 700 kb/s. In a case of multichannel registration of EEG one observes 32 - 128 electrodes. Even if the sampling rate is 200 Hz per electrode and resolution of 16 bits then one is capable to transfer 128 channels with identification over the isolation ISOLATION. One feature of a Bluetooth system is that it generates a piconetwork, presently seven units may be connected to the same network. A piconetwork may further be connected to other piconetworks. In this way a system may be expanded in a flexible manner to cover all practical situations. Networks may be generated also by other means and one possibility is a generation of JAVA based networks using the JINI principle. This system is developed among others by Sun Microsystems Inc., USA.

In general a system of the invention may be connected to a data network such as to Internet.

In this case one may control operations of various blocks, e.g. A1, AN, MUX, BT, CU, TR3, TRI1, TRI2, SC1 and SC2, via a data network and set functional parameters such as gains of amplifiers, e.g. A1 and AN, and parameters of filters which may be included by signal conditioning unit SC1, SC2. In that case, e.g. a distant nurse, who notices that one of ECG electrodes is loose, may disconnect an amplifier or change its gain via data network using for example WAP protocol based control. In this case the system of invention enables a full mobility of personnel and gives a possibility to monitor patients and change the operation of the system of the invention as necessary.

For power and signal transfer it is beneficial that output and return currents flow close each other. In other words the signal and return currents are close each other in transmission line and correspondingly the currents and return currents of power supply flow close each other. Hence, noise currents from object P are flowing as common mode currents. By addition of some material on transmission line to increase the impedance for common mode current one may advantageously add the impedance of path of noise currents. Such proper materials are among others ferrites, which are manufactured among others by Philips, Nederland. One may also add ferrite in some part of a jacket of a cable of transmission line TL by mixing it in insulation of the cable. Ferrite material may used only in a limited manner near a magnetic resonance imaging device, but most other applications of the invention does not have this kind of limitation. FIG 5 shows an example of a use of ferrite FER. Impedance for common mode currents may also be increased by adding inductance of a transmission line by winding from the cable a coil with an air core.

It should be mentioned that the amplifiers AMPLIFIERS may be situated in an immediate neighborhood of the electrodes ELECTRODES, then one reduces coupling of interferences on the connections between electrodes and amplifiers. Materials used with magnetic resonance imaging must be as weakly magnetized as possible. This is because magnetized material generates inhomogeneities of magnetic field and in this way generates distortion of a final image.

During an operation of a system the transmission of PA can be made as pulsed and between these pulses information transmission from registration units TRJ1 and TRJ2 takes place. The transmission of these may also take place in such a way that at each moment only a limited number, for example one, registration unit is active. The timing can be controlled by REGISTRATION unit or an active unit reserves a transmission line TL1, TL2, TLS for its use during transmission and the following units TRJ wait release of the transmission line.

For this purpose there are memory means, such as semiconductor memory, FLASH memory or corresponding device, in a registration unit. Often it is feasible to keep the output level of PA constant and bandwidth narrow and advantageously outside the used signal band. It may also possible that RT1 and RT2 contain some energy storage such as a supercapacitor, which supplies energy for transmission for RT1 and RT2 and these send a signal to PA for request to send more power, when the energy content of the energy storage has decreased below some certain limit. The energy content of the energy storage may be find out by utilizing terminal voltage of the capacitor acting as an energy storage.

FIG 8 shows isolation ISOLATION, which has been realized using a coil L1 and capacitor C1 in a serial connection. In addition to this there has been coupled parallel to L1 a pair of diodes D 1 and a capacitor C2 in such a way that at high level, when D 1 is conductive, C2 and L1 form a high impedance parallel resonance circuit and in such a way prevents a progress of a signal at this frequency through ISOLATION or at least attenuates intensity of signal. If D is omitted above described dependence on signal level does not exist.

FIG 9 shows as an example an instrument for studies of blood vessels, such as a catheter with a detection unit situated at the tip TIP, there is isolation ISOLATION, power means D1 and C1, signal conditioning means SC, such as an amplifier and a transducer TRANS such as a NMR coil. The body CAT serves as a transmission line, which may be composed of several capacitively coupled segments for improvement of isolation. Along catheter CAT one may also supply energy for improvement of visibility of the tip of the catheter during magnetic resonance imaging using Overhauser phenomenon. Here one may utilize technique, which has been described in the reference Sepponen US 5211166. Also one may utilize electron spin resonance for localization of some part of the instrument. In that case CAT and TIP include the necessary means, which are described in the reference and later publications based thereon. CAT may be also a part of signal means used with magnetic resonance imaging, such as a part of a surface coil or an endocoil.

TIP may also include image generation means such as CCD of CMOS transducer. Then it may be used for example for endoscopy or for intraoral X-ray imaging. TIP as well as other registration units may include also operational means such as a laser diode or a drill for removal of thrombus of a blood vessel. Other possible alternatives include means for generation of Overhauser phenomenon or for detection of electron spin resonance. Furthermore, operational means may include lightning means such as e.g. a light diode. Operational means may also include means for application of hyperthermia therapy. The power for operational means is conducted through isolation according to the invention.

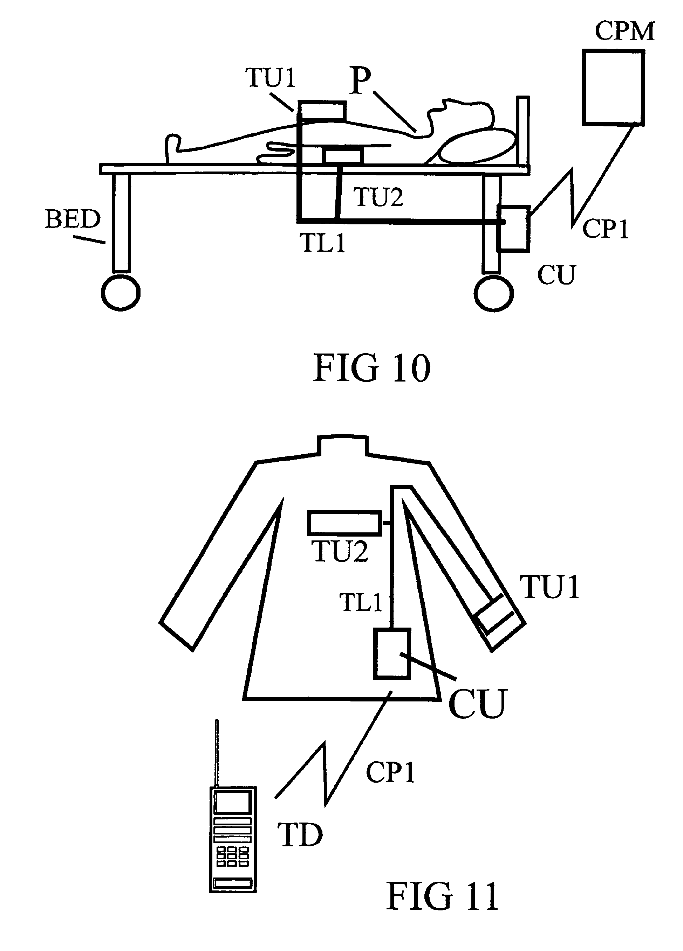

FIG 10 shows a patient bed BED having a frame in which means of the invention CU, TL1 and transducer units TU1 and TU2 has been embedded. TU1 and TU2 can be e.g. transducer units for ECG and blood oxygen saturation level. CU has been connected in a desired way wirelessly or with a wire via communication path CP1 to central patient monitoring unit CPM, where one has installed needed alarm, signal transfer, etc. functions. One advantageous mode of invention has been described in the following. According to the invention transducers TU1 and TU2 may be practically connected to CU, which when the bed is staying still may be connected wirelessly to the local area network of the hospital and during this time the energy will be obtained from mains outlet. When the bed is being moved the energy will be obtained from the battery unit of CU. A system of the invention may be advantageously built in frames of devices, equipment and rooms. Such are e.g. anesthesia machines, ambulances, cars, operational theatres, medical imaging devices, and industrial robots, which are used for quality control and associated electronic measurements.

As an example FIG 11 shows clothing, which may be e.g. protective clothing of a fireman, diving suit, sport clothing, clothing designed for harsh climates or clothing for research purposes. There is central unit CU placed in clothing; CU includes a battery or another type of power source such as e.g. a solar cell.

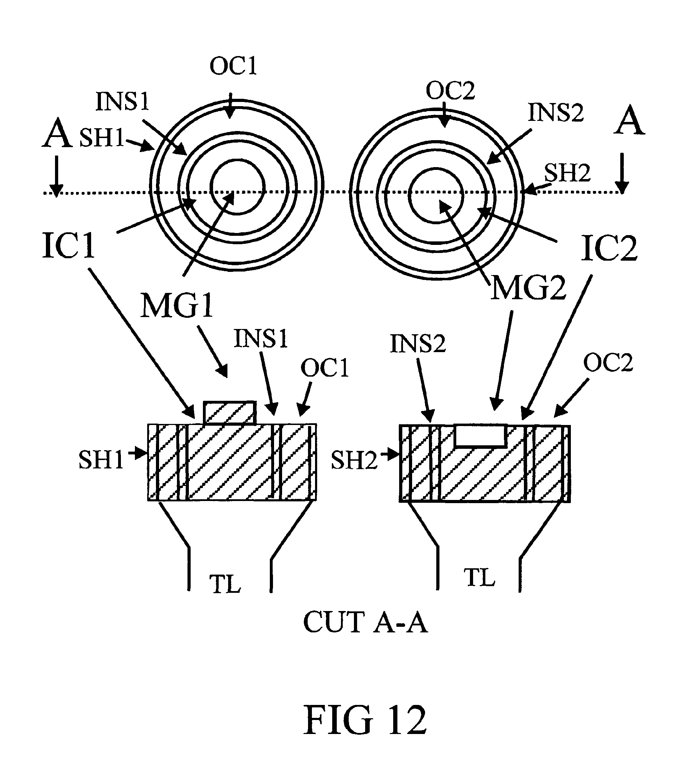

A system must offer a solution, which enables fast connection and disconnection of several transducers. One possible solution is depicted in FIG 12. In the shown solution there is on a transmission line TL a connector including two parts, which may be connected together. In the connector the male part is composed from ferromagnetic middle part MG1, inner conductor IC1, insulation INS1, which is between IC 1 and outer conductor OC 1 and from shielding layer SH1. FIG 12 shows top views of the parts of the connector (above) and intersection side views (below). Correspondingly a female part is composed from a magnet part MG2, and also inner conductor IC2, insulation INS2, outer conductor OC2 and shield SH2. This connector works in such a way that MG1 and MG2, from which at least one includes permanent magnet material, attract during connection each other, then conductors of the connector IC1, IC2, and OC1, OC2 make conductive or capacitive contacts. A contact may also include both conductive and capacitive components simultaneously. This type of connector may be used e.g. for connecting heart rate transducer to a system of for connecting a transducer of pressure of a gas bottle. A connector may be equipped with retaining means, such as screw fixation, bayonet fixation, Velcro strap or with various type of bail solution. In arrangements of the invention one may use various types of connectors, which fulfill the need at each time as well as possible.

According to the invention transducer and central units may be addressed using Internet protocol and be controlled using this protocol. For this purpose the units have necessary means. The WAP-protocol used with mobile devices is also possible and in parallel with this one may use IP-protocol. The invention is not limited to said protocols but also other open or closed and also application specific protocols may be used with an arrangement of the invention.

Although the examples described above are related to medical technology, a solution of the invention may be applied in many equipment related problems. For example induction heaters, high voltage devices, such as X-ray devices, electric motors, cars, airplanes etc. include instrumentation systems, where an arrangement of invention may be used to decisively improve performance of operations.

Above is only illustrated only some of possible realizations of the invention. The invention is not restricted to the above, but it can be utilized in many other accomplishments within the limits of the inventive thought as in the enclosed patent claims.

## Claims

1. An arrangement for registration of signals, such as EEG and ECG, from an object (P) using transducers (T1, T2) in noisy environments, such as in airplane, car, industrial establishment, magnetic resonance imaging unit, intensive care unit and operating theatre, where other significant electromagnetic energy appears, which is being coupled to the arrangement of registration and which considering use of the signals decreases their utility value or which by coupling rises possibility for damaging of the object, the arrangement including a transmission line or path (TLS, TL1,TL2) for transferring the registered signals to a central unit (CU) and a signal conditioning means and connection means (SC, TRI1, TRI2) between the transducers (T1, T2) and the transmission line or path (TLS, TL1, TL2), the transducers (T1, T2) and the signal conditioning means and connection means (SC, TRI1, TRI2) being supplied with driving energy via the transmission line (TLS, TL1, TL2) **characterized in that**, said transfer of driving energy takes place via at least one isolation means (ISOLATION) which are part of the connection means (TRI1, TRI2) and/or part of the transmission line (TLS, TL1, TL2) and are adapted for reducing the coupling of said other electromagnetic energy, said transfer of driving energy taking place at a radio frequency which is at least one decade higher than that of the signals to be registered and that of said other electromagnetic energy.

2. An arrangement as set forth in claim 1 **characterized in that** at least part of needed isolation takes place in the transmission line (TLS, TL1, TL2) in such a way that the isolation means (ISOLATION) includes at least one coupling element, which transfer electromagnetic energy via electromagnetic field, such as for example capacitive (C, C1) or inductive coupling element.

3. An arrangement as set forth in any of the claims above **characterized in that** the isolation means (ISOLATION) include a capacitive coupling element (C, C1), and additionally an element for reduction of coupling impedance, such as for example an inductive element (L, L1), which is coupled in series with said capacitive element (C1), and eventually in parallel with said inductive element (L1) there is another capacitive element (C2), which generates parallel resonance with said inductive element (L1) at frequency, which is one of interference frequencies appearing in environment, such as excitation frequency of a magnetic resonance imaging device or operating frequency of an electric knife.

4. An arrangement as set forth in any of the claims above **characterized in that** to the transmission line (TL, TLS) there is attached means (SPL1, SPL2) for dividing transmission line to two or more transmission lines (TL1, TL2).

5. An arrangement as set forth in any of the claims above **characterized in that** transfer of registered signals to a central unit (CU, TR3) takes place via the transmission path (TL2) and this transmission path uses wireless communication, such as for example radio, magnetic field, ultrasound or infrared communication.

6. An arrangement as set forth in the claims 1 - 4 **characterized in that** transfer of registered signals to a central unit (CU, TR3) takes place via the same transmission path as the power transmission for signal conditioning means and connection means (SC, RT1, RT2) and advantageously via the same isolation means (ISOLATION).

7. An arrangement as set forth in any of the claims above **characterized in that** operation of a power means (POSC, PA) of the arrangement is time wise discontinuous.

8. An arrangement as set forth in any of the claims above **characterized in that** the arrangement includes for storage of information memory means, such as semiconductor memory, for example Flash memory.

9. An arrangement as set forth in any of the claims above **characterized in that** signal is modulated by the signal conditioning means and connection means (SC, TRI1, TRI2) in such a way, that needed reliability, and signal bandwidth is achieved using one or some of following methods for modulation: amplitude, frequency, phase, spread spectrum, OFDM and ultra wide bandwidth modulation.

10. An arrangement as set forth in any of the claims above **characterized in that** the central unit (CU) or the signal conditioning means and connection means (SC, TRI1, TRI2) is connected to at least one data network such as piconetwork according to the Bluetooth standard or Internet.

11. An arrangement as set forth in any of the claims above **characterized in that** the central unit (CU) or the signal conditioning means and connection means (SC, TRI1, TRI2) may be controlled and properties of operation, such as for example gain or bandwidth, may be changed via data network, local or wide area network, such as piconetwork according to Bluetooth standard or Internet.

12. An arrangement as set forth in any of the claims above **characterized in that** at least part of it is placed in means to be carried by person or animal or otherwise intimately associated, such as clothing (CL), diving means, protection means or patient bed (BED) and it includes one or several transducer means (TU1, TU2) for registering information from one or some of following: human body, animal body (P), a device to be carried with, such as for example gas bottle, and environment.

13. An arrangement as set forth in any of the claims above **characterized in that** it is used for monitoring physiologic signals from person (P) under medical diagnostic or therapeutic activity, such as surgical operation, intensive care or magnetic resonance imaging.

14. An arrangement as set forth in any of the claims above **characterized in that** registration units such as for example amplifiers (AMPLIFIERS) are in vicinity of the transducers (T1, T2) such as electrodes (ELECTRODES) and when circumstance so requires, for example in vicinity of magnetic resonance imaging device, are realized using one or some of following techniques: flip-chip, gluing and bonding.

15. An arrangement as set forth in any of the claims above **characterized in that** a central unit (CU) of the arrangement is connected via a wired or wireless transmission path (CP1) to some means (CPM, TD) of other system such as a patient monitoring system or a telephone network.

16. An arrangement as set forth in any of the claims above **characterized in that** at least one of it's units may be controlled using commands according to protocol, for example WAP-protocol, of connected network utilizing terminal device (TD), for example mobile phone, wrist computer, such as diving computer or means of some other system such as means (CPM) of patient monitoring system.

17. An arrangement as set forth in any of the claims above **characterized in that** at least in some part of the transmission line (TL, TL 1, TL2) there is one or several connector (CON) including contacting surfaces for contacting conductive parts (IC 1, IC2, OC1, OC2) of the trans-mission line and means for securing connection (MG1, MG2) such as for example magnet, thread, bail, bayonet or Velcro tape.

18. An arrangement as set forth in any of the claims above **characterized in that** the transmission line (TL) is integral part of essentially compliant structure, such as for example catheter (CAT) or signals coil means of magnetic resonance imaging device, such as surface or endoscopic coil, which includes at least one part (TIP) including means (TRANS) for detecting signal to be registered, such as for example NMR signal and for processing of signal (SC) and for transferring over isolation (ISOLATION) and necessary power means (D1, C1).

19. An arrangement as set forth in any of the claims above **characterized in that** the arrangement includes means for collecting image information, such as for example CMOS or CCD circuit

20. An arrangement as set forth in any of the claims above **characterized in that** the arrangement is used for X-ray imaging, such as for X-ray imaging of dentition or joints.

21. An arrangement as set forth in any of the claims above **characterized in that** the arrangement includes one or several operational means, such as laser or drill means, means for generation of Overhauser phenomena, means for detection of electron spin resonance, means for hyperthermia or means for lighting.

## Patentansprüche

1. Anordnung zur Erfassung von Signalen, wie EEG und EKG, von einem Objekt (P), unter Verwendung von Übertragern (T1, T2) in lauten Umgebungen, wie im Flugzeug, Auto, industrieller Einrichtung, Magnetresonanz-Bildgebungseinheit, Intensivpflege-Einheit, und Operationssaal, wo andere signifikante elektromagnetische Energie auftritt, die gekoppelt wird mit der Erfassungsanordnung und die bei Betrachtung der Verwendung der Signale ihren Nutzwert verringert oder die durch Kopplung die Möglichkeit erhöht, das Objekt zu beschädigen, wobei die Anordnung eine Übertragungsleitung oder einen Übertragungspfad (TLS, TL1, TL2) umfasst zur Übertragung der erfassten Signale zu einer Zentraleinheit (CU) und ein Signalbearbeitungsmittel und Verbindungsmittel (SC, TRI1, TRI2) zwischen den Übertragern (T1, T2) und der Übertragungsleitung oder dem Übertragungspfad (TLS, TL1, TL2), wobei die Übertrager (T1, T2) und das Signalbearbeitungsmittel und Verbindungsmittel (SC, TRI1, TRI2) mit Antriebsenergie durch die Übertragungsleitung (TLS, TL1, TL2) versorgt werden,
**dadurch gekennzeichnet,**
**dass** die genannte Übertragung von Antriebsenergie mittels mindestens eines Isolationsmittels (ISOLATION) erfolgt, das Teil der Verbindungsmittel (TRI1, TRI2) und/oder Teil der Übertragungsleitung (TLS, TL1, TL2) ist und angepasst ist oder wird zur Reduzierung der Kopplung der genannten anderen elektromagnetischen Energie, wobei die genannte Übertragung von Antriebsenergie mit einer Funkfrequenz stattfindet, die zumindest eine Dekade höher ist als die der zu erfassenden Signale und die der genannten anderen elektromagnetischen Energie.

2. Anordnung wie in Anspruch 1 dargelegt,
**dadurch gekennzeichnet,**
**dass** zumindest ein Teil der erforderlichen Isolation in der Übertragungsleitung (TLS, TL1, TL2) erfolgt, derart, dass das Isolationsmittel (ISOLATION) zumindest ein Kopplungselement umfasst, das elektromagnetische Energie mittels eines elektromagnetischen Feldes überträgt, wie beispielsweise ein kapazitives (C, C1) oder induktives Kopplungselement.

3. Anordnung wie in irgendeinem der vorstehenden Ansprüche dargelegt,
**dadurch gekennzeichnet,**
**dass** das Isolationsmittel (ISOLATION) ein kapazitives Kopplungselement (C, C1) umfasst, und zusätzlich ein Element zur Reduzierung von Kopplungsimpedanz, wie beispielsweise ein induktives Element (L, L1), das in Reihe gekoppelt ist oder wird mit dem genannten kapazitiven Element (C1), und schließlich gibt es parallel zu dem genannten induktiven Element (L1) ein weiteres kapazitives Element (C2), das eine Parallelresonanz mit dem genannten induktiven Element (L1) erzeugt bei einer Frequenz, die eine der Interferenzfrequenzen ist, die in der Umgebung auftreten, wie eine Erregungsfrequenz einer Magnetresonanz-Bildgebungsvorrichtung oder eine Arbeitsfrequenz eines elektrischen Messers.

4. Anordnung wie in irgendeinem der vorstehenden Ansprüche dargelegt,
**dadurch gekennzeichnet,**
**dass** an die Übertragungsleitung (TL, TLS) ein Mittel (SPL1, SPL2) angeschlossen ist oder wird zur Aufteilung von Übertragungsleitung in zwei oder mehr Übertragungsleitungen (TL1, TL2).

5. Anordnung wie in irgendeinem der vorstehenden Ansprüche dargelegt,
**dadurch gekennzeichnet,**
**dass** die Übertragung von erfassten Signalen zu einer Zentraleinheit (CU, TR3) mittels des Übertragungspfads (TL2) erfolgt und dass dieser Übertragungspfad drahtlose Kommunikation verwendet, wie beispielsweise Funk-, Magnetfeld-, Ultraschall- oder Infrarotkommunikation.

6. Anordnung wie in den Ansprüchen 1 bis 4 dargelegt,
**dadurch gekennzeichnet,**
**dass** die Übertragung von erfassten Signalen zu einer Zentraleinheit (CU, TR3) mittels des gleichen Übertragungspfads erfolgt wie die Energieübertragung für Signalbearbeitungsmittel und Verbindungsmittel (SC, RT1, RT2) und vorzugsweise mittels des gleichen Isolationsmittels (ISOLATION).

7. Anordnung wie in irgendeinem der vorstehenden Ansprüche dargelegt,
**dadurch gekennzeichnet,**
**dass** der Betrieb eines Energiemittels (POSC, PA) der Anordnung zeitweise diskontinuierlich erfolgt.

8. Anordnung wie in irgendeinem der vorstehenden Ansprüche dargelegt,
**dadurch gekennzeichnet,**
**dass** die Anordnung zur Speicherung von Information Speichermittel umfasst, wie Halbleiterspeicher, beispielsweise Flash-Speicher.

9. Anordnung wie in irgendeinem der vorstehenden Ansprüche dargelegt,
**dadurch gekennzeichnet,**
**dass** das Signal durch die Signalbearbeitungsmittel und Verbindungsmittel (SC, TRI1, TRI2) derart moduliert ist oder wird, dass erforderliche Zuverlässigkeit und Signalbandbreite durch eine oder mehrere der folgenden Modulationsmethoden erreicht ist oder wird: Amplituden-, Frequenz-, Phasen-, Bandbreiten-, OFDM- und Ultrabreitbandmodulation.

10. Anordnung wie in irgendeinem der vorstehenden Ansprüche dargelegt,
**dadurch gekennzeichnet,**
**dass** die Zentraleinheit (CU) oder die Signalbearbeitungsmittel und Verbindungsmittel (SC, TRI1, TRI2) zu zumindest einem Datennetzwerk wie Piconet entsprechend dem Bluetooth-Standard oder Internet verbunden ist oder wird.

11. Anordnung wie in irgendeinem der vorstehenden Ansprüche dargelegt,
**dadurch gekennzeichnet,**
**dass** die Zentraleinheit (CU) oder die Signalbearbeitungsmittel und Verbindungsmittel (SC, TRI1, TRI2) steuerbar sind und Eigenschaften des Betriebs, wie beispielsweise Verstärkung oder Bandbreite, verändert werden können mittels Datennetzwerk, lokalem oder großräumigem Netzwerk, wie Piconet, entsprechend dem Bluetooth-Standard oder Internet.

12. Anordnung wie in irgendeinem der vorstehenden Ansprüche dargelegt,
**dadurch gekennzeichnet,**
**dass** zumindest ein Teil davon in einem von einer Person oder einem Lebewesen zu tragenden oder in anderer Weise von vornherein verbundenen Mittel platziert ist oder wird, wie Kleidung (CL), Tauchmittel, Schutzmittel oder Patientenbett (BETT), und dass sie oder es ein oder mehrere Übertragungsmittel (TU1, TU2) zur Informationserfassung von einem oder einigen der folgenden umfasst: menschlicher Körper, Tierkörper (P), eine mit sich zu tragende Vorrichtung, wie beispielsweise Gasflasche, und Umgebung.

13. Anordnung wie in irgendeinem der obigen Ansprüche dargelegt,
**dadurch gekennzeichnet,**
**dass** sie zur Überwachung von physiologischen Signalen von einer Person (P) während einer medizinisch-diagnostischen oder therapeutischen Aktivität dient, wie eine chirurgische Tätigkeit, Intensivpflege oder Magnetresonanzbildgebung.

14. Anordnung wie in irgendeinem der vorstehenden Ansprüche dargelegt,
**dadurch gekennzeichnet,**
**dass** die Erfassungseinheiten, wie beispielsweise Verstärker (VERSTÄRKER), in Nachbarschaft der Übertrager (T1, T2) wie Elektroden (ELEKTRODEN) sind und wenn es der Umstand so erfordert, beispielsweise in Nachbarschaft von einer Magnetresonanz-Bildgebungseinheit, umgesetzt sind oder werden unter Verwendung einer oder einiger der folgenden Techniken: Flip-Chip, Kleben und Verbinden.

15. Anordnung wie in irgendeinem der vorstehenden Ansprüche dargelegt,
**dadurch gekennzeichnet,**
**dass** eine Zentraleinheit (CU) der Anordnung mittels eines drahtgebundenen oder drahtlosen Übertragungspfads (CP1) verbunden ist mit Mitteln (CPM, TD) eines anderen Systems wie einem Patientenüberwachungssystem oder einem Telefonnetzwerk.

16. Anordnung wie in irgendeinem der vorstehenden Ansprüche dargelegt,
**dadurch gekennzeichnet,**
**dass** zumindest eine von deren Einheiten gesteuert werden kann unter Benutzung von Kommandos entsprechend einem Protokoll, beispielsweise eines WAP-Protokolls, von verbundenem Netzwerk unter Verwendung einer Eingabevorrichtung (TD), beispielsweise Mobiltelefon, Handgelenkscomputer, wie Tauchcomputer, oder Mittel eines anderen Systems, wie Mittel (CPM) eines Patientenüberwachungssystems.

17. Anordnung wie in irgendeinem der vorstehenden Ansprüche dargelegt,
**dadurch gekennzeichnet,**
**dass** zumindest in einem Teil der Übertragungsleitung (TL, TL1, TL2) ein oder mehrere Verbinder (CON) sind, umfassend Kontaktflächen zur Kontaktierung leitender Teile (IC1, IC2, OC1, OC2) der Übertragungsleitung und Mittel zur Sicherung der Verbindung (MG1, MG2), wie beispielsweise Magnet, Faden, Bügel, Bajonett oder Klettband.

18. Anordnung wie in irgendeinem der vorstehenden Ansprüche dargelegt,
**dadurch gekennzeichnet,**
**dass** die Übertragungsleitung (TL) integraler Teil von einer im wesentlichen nachgiebigen Struktur, wie beispielsweise Katheder (CAT) oder Signalspulmittel von einer Magnetresonanz-Bildgebungseinheit, wie Oberflächen- oder endoskopische Spule, die zumindest ein Teil (TIP) umfasst, das Mittel (TRANS) zur Erkennung von zu erfassenden Signalen umfasst, wie beispielsweise ein NMR-Signal und zur Signalverarbeitung (SC) und zur Übertragung über die Isolation (ISOLATION) und notwendige Energiemittel (D1, C1).

19. Anordnung wie in irgendeinem der vorstehenden Ansprüche dargelegt,
**dadurch gekennzeichnet,**
**dass** die Anordnung Mittel zum Sammeln von Bildinformation umfasst, wie beispielsweise CMOS- oder CCD-Schaltkreis.

20. Anordnung wie in irgendeinem der vorstehenden Ansprüche dargelegt,
**dadurch gekennzeichnet,**
**dass** die Anordnung zur Röntgenbildgebung, wie zur Röntgenbildgebung von Zähnen oder Gelenken, verwendet wird.

21. Anordnung wie in irgendeinem der vorstehenden Ansprüche dargelegt,
**dadurch gekennzeichnet,**
**dass** die Anordnung eines oder mehrere Betriebsmittel umfasst, wie Laser- oder Bohrmittel, Mittel zur Erzeugung des Overhauser-Phänomens, Mittel zur Erzeugung von Elektronenspinnresonanz, Mittel für Hyperthermie oder Mittel zur Beleuchtung.

## Revendications

1. Agencement pour enregistrement de signaux, tels que EEG et ECG, à partir d'un objet (P) en utilisant des transducteurs (T1, T2) dans des environnements bruyants, tels que dans un aéronef, une voiture, un établissement industriel, une unité d'imagerie par résonance magnétique, une unité de soins intensifs et une salle d'opération, où une autre énergie électromagnétique significative apparaît, qui est couplée à l'agencement d'enregistrement et qui, en considérant l'utilisation des signaux, diminue leur valeur d'utilité ou qui, par couplage, élève la possibilité d'endommagement de l'objet, l'agencement comprenant une ligne ou un trajet de transmission (TLS, TL1, TL2) destiné à transférer les signaux enregistrés vers une unité centrale (UC) et un moyen de conditionnement de signal et un moyen de connexion (SC, TRI1, TRI2), entre les transducteurs (T1, T2) et la ligne ou le trajet de transmission (TLS, TL1, TL2), les transducteurs (T1, T2) et le moyen de conditionnement de signal et le moyen de connexion (SC, TRI1, TRI2) étant alimentés en une énergie de fonctionnement via la ligne de transmission (TLS, TL1, TL2), **caractérisé en ce que** ledit transfert d'énergie de fonctionnement a lieu via au moins un moyen d'isolation (ISOLATION) qui est une partie du moyen de connexion (TRI1, TRI2), et/ou une partie de la ligne de transmission (TLS, TL1, TL2) et est adapté pour réduire le couplage de ladite autre énergie électromagnétique, ledit transfert d'énergie de fonctionnement ayant lieu à une radiofréquence qui est au moins une décade plus élevée que celle des signaux à enregistrer et celle de ladite autre énergie électromagnétique.

2. Agencement selon la revendication 1, **caractérisé en ce qu'**au moins une partie de l'isolation nécessaire a lieu dans la ligne de transmission (TLS, TL1, TL2) de telle manière que le moyen d'isolation (ISOLATION) comprend au moins un élément de couplage, qui transfère l'énergie électromagnétique via un champ électromagnétique, tel que par exemple, un élément de couplage capacitif (C, C1) ou inductif.

3. Agencement selon l'une quelconque des revendications précédentes, **caractérisé en ce que** le moyen d'isolation (ISOLATION) comprend un élément de couplage capacitif (C, C1) et, en outre, un élément pour réduction d'impédance de couplage, tel que, par exemple, un élément inductif (L, L1), qui est couplé en série avec ledit élément capacitif (C1), et éventuellement en parallèle avec ledit élément inductif (L1), il existe un autre élément capacitif (C2), qui génère une résonance parallèle avec ledit élément inductif (L1) à une fréquence, qui est l'une des fréquences d'interférence apparaissant dans l'environnement, telle qu'une fréquence d'excitation d'un dispositif d'imagerie par résonance magnétique ou une fréquence de fonctionnement d'un couteau électrique.

4. Agencement selon l'une quelconque des revendications précédentes, **caractérisé en ce qu'** à la ligne de transmission (TL, TLS), est associé un moyen (SPL1, SPL2) destiné à diviser la ligne de transmission en deux ou plusieurs lignes de transmission (TL1, TL2).

5. Agencement selon l'une quelconque des revendications précédentes, **caractérisé en ce que** le transfert de signaux enregistrés vers une unité centrale (CU, TR3) a lieu via le trajet de transmission (TL2) et ce trajet de transmission utilise une communication sans fil, telle que par exemple une communication radio, par champ magnétique, ultrasonore ou infrarouge.

6. Agencement selon les revendications 1 à 4, **caractérisé en ce que** le transfert de signaux enregistrés vers une unité centrale (CU, TR3) a lieu via le même trajet de transmission que la transmission de puissance pour le moyen de conditionnement du signal et le moyen de connexion (SC, RT1, RT2) et avantageusement via le même moyen d'isolation (ISOLATION).

7. Agencement selon l'une quelconque des revendications précédentes, **caractérisé en ce que** le fonctionnement d'un moyen de puissance (POSC, PA) de l'agencement est discontinu dans le temps.

8. Agencement selon l'une quelconque des revendications précédentes, **caractérisé en ce que** l'agencement comprend, pour le stockage d'informations, des moyens de mémoire tels qu'une mémoire semi-conductrice, par exemple une mémoire Flash.

9. Agencement selon l'une quelconque des revendications précédentes, **caractérisé en ce que** le signal est modulé par le moyen de conditionnement de signal et le moyen de connexion (SC, TR11, TR12) de telle manière qu'une fiabilité nécessaire et une largeur de bande de signal sont obtenues en utilisant l'un des procédés suivants pour la modulation : modulation d'amplitude, de fréquence, de phase, à spectre étalé, OFDM et à largeur de bande ultra-large.

10. Agencement selon l'une quelconque des revendications précédentes, **caractérisé en ce que** l'unité centrale (UC) ou le moyen de conditionnement de signal et le moyen de connexion (SC, TRI1, TRI2) sont connectés à au moins un réseau de données tel qu'un pico-réseau selon la norme Bluetooth ou Internet.

11. Agencement selon l'une quelconque des revendications précédentes, **caractérisé en ce que** l'unité centrale (UC) ou le moyen de conditionnement de signal et le moyen de connexion (SC, TRI1, TRI2) peuvent être commandés et des propriétés de fonctionnement, telles que par exemple le gain ou la largeur de bande, peuvent être changées via un réseau de données, un réseau de zones locales ou larges, tel qu'un pico-réseau selon la norme Bluetooth ou Internet.

12. Agencement selon l'une quelconque des revendications précédentes, **caractérisé en ce qu'**au moins une partie de celui-ci est placée dans un moyen à porter par une personne ou un animal ou sinon qui lui est intimement associé, tel qu'un vêtement (CL), un moyen de plongée, un moyen de protection ou un lit de patient (BED) et **en ce qu'**il comprend un ou plusieurs moyens transducteurs (TU1, TU2) pour enregistrer des informations à partir d'un ou plusieurs des éléments suivants : corps humain, corps animal (P), un dispositif à porter, tel que, par exemple, une bouteille de gaz et l'environnement.

13. Agencement selon l'une quelconque des revendications précédentes, **caractérisé en ce qu'**il est utilisé pour surveiller les signaux physiologiques provenant d'une personne (P) sous diagnostic médical ou sous activité thérapeutique, tels qu'une opération chirurgicale, un soin intensif ou une imagerie par résonance magnétique.

14. Agencement selon l'une quelconque des revendications précédentes, **caractérisé en ce que** les unités d'enregistrement telles que, par exemple, des amplificateurs (AMPLIFICATEURS) sont au voisinage des transducteurs (T1, T2) tels que des électrodes (ELECTRODES) et, selon les circonstances, par exemple au voisinage d'un dispositif d'imagerie par résonance magnétique, sont réalisées en utilisant une ou plusieurs des techniques suivantes : puce retournée, collage et liaison.

15. Agencement selon l'une quelconque des revendications précédentes, **caractérisé en ce qu'**une unité centrale (UC) de l'agencement est connectée via un trajet de transmission à fil ou sans fil (CP1) à certains moyens (CPM, TD) d'un autre système tel qu'un système de surveillance de patient ou un réseau téléphonique.

16. Agencement selon l'une quelconque des revendications précédentes, **caractérisé en ce qu'**au moins une de ses unités peut être commandée en utilisant des ordres selon un protocole, par exemple un protocole WAP, d'un réseau connecté utilisant un dispositif terminal (TD), par exemple un téléphone mobile, un ordinateur de poignet, tel qu'un ordinateur de plongée ou un moyen de quelque autre système tel qu'un moyen (CPM) de système de surveillance de patient.

17. Agencement selon l'une quelconque des revendications précédentes, **caractérisé en ce qu'**au moins dans une certaine partie de la ligne de transmission (TL, TL1, TL2), il existe un ou plusieurs connecteurs (CON) comprenant des surfaces de contact prévues pour venir en contact avec des parties conductrices (IC1, IC2, OC1, OC2) de la ligne de transmission et un moyen pour fixer une connexion (MG1, MG2) tel que, par exemple, un aimant, un filetage, un étrier, une baïonnette ou une bande Velcro.

18. Agencement selon l'une quelconque des revendications précédentes, **caractérisé en ce que** la ligne de transmission (TL) est une partie solidaire d'une structure essentiellement adaptable, telle que par exemple un cathéter (CAT) ou un moyen formant bobine de signaux d'un dispositif d'imagerie par résonance magnétique, tel qu'une bobine de surface ou endoscopique, qui comprend au moins une partie (TIP) comprenant un moyen (TRANS) destiné à détecter un signal à enregistrer, tel que, par exemple, un signal RMN et destiné à traiter un signal (SC) et à le transférer sur isolation (ISOLATION) et un moyen de puissance nécessaire (D1, C1).

19. Agencement selon l'une quelconque des revendications précédentes, **caractérisé en ce que** l'agencement comprend un moyen destiné à collecter des informations d'image, tel que, par exemple, un circuit CMOS ou CCD.

20. Agencement selon l'une quelconque des revendications précédentes, **caractérisé en ce que** l'agencement est utilisé pour une imagerie par rayons X, telle qu'une imagerie par rayons X de la dentition ou des articulations.

21. Agencement selon l'une quelconque des revendications précédentes, **caractérisé en ce que** l'agencement comprend un ou plusieurs moyens opérationnels, tels qu'un laser ou un moyen de forage, un moyen pour la génération du phénomène Overhauser, un moyen de détection de résonance paramagnétique des électrons, un moyen d'hyperthermie ou un moyen d'éclairage.
